# EUROPEAN PATENT SPECIFICATION

(11) **EP 1 366 781 B1**
(45) Date of publication and mention of the grant of the patent: **13.12.2006**
(21) Application number: 02011946.7
(22) Date of filing: 29.05.2002
(51) Int. Cl.: A61N 2/02

(54) **Apparatus for treating a body part using an electromagnetic field**
Vorrichtung zur Behandlung eines menschlichen Körperteils mittels eines elektromagnetischen Feldes
Appareil pour traiter une partie du corps humain avec un champ électromagnétique

(43) Date of publication of application: 03.12.2003
(73) Proprietor: Markoll, Richard, Dr., 81371 München (DE)
(72) Inventor: Markoll, Richard, Dr., 81371 München (DE)
(74) Representative: HOFFMANN EITLE

(56) References cited:
- EP-A- 0 824 025
- WO-A-01/15770
- US-A- 5 131 904

## Description

### Technical field

The present invention relates generally to an apparatus for treating a body part using an electromagnetic field and more particularly to an apparatus according to the preamble of claim 1.

An electromagnetic field can be applied to a body part to alter, arrest, or heal diseases and conditions including painful, degenerative, injurious, or inflammatory conditions of body parts, or to relieve pain or other uncomfortable or unwanted sensations associated with these named diseases and conditions.

### Related background art

It has been recognized in the prior art that the application of a magnetic field to diseased body parts can in some way improve the condition.

US 5,131,904 discloses a process for treating an arthritic body organ, the process being performed in the absence of any electrical field and including the step of subjecting the arthritic body organ to an electromagnetic field of under 20 Gauss and generated by an annular coil into the center of which the arthritic body organ is placed, the coil being driven by a pulsed DC voltage having a rectangular wave form consisting of an abruptly rising and abruptly deteriorating current pulsing at the rate of 1 to 30 pulse bursts per second. In this prior art apparatus, the target body part may be supported to be in position eccentric to the central flux portion of the magnetic field within the coil. This can be accomplished by a shapeable pillow which assists the proper positioning of the body part.

The therapy carried out by means of such an apparatus is now known as PST (pulse signal therapy). Directing a specific time frequency and wave form, of low amplitude magnetic field into and onto a target body part in an almost axial arrangement for an extended specified length of time, allows or even causes the electron or ionic flow to remain in either a plus or minus state. This action does stimulate the electrical potential of the body part. Thus a regeneration process is initiated by which the cells are able to and will more fully perform their intended genetic functions.

In an apparatus developed by "PST Pulsierende Signal Therapie GmbH", the patient places the body part to be treated (in particular his arm, leg or torso) inside a cylindrical coil in which pulse signals are generated. The intensity and frequency of these pulse signals are varied according to a predetermined biological pattern, depending on what kind of disease or condition is to be treated. The pulse signals have a relatively low frequency and energy. To assists the proper positioning of the body part inside the coil, a shapeable pillow may be provided also in this apparatus.

However, during the course of treatment, the apparatus is used repeatedly and with intervals, e.g. for about 30 minutes every 24 to 48 hours. In the known apparatuses, it is difficult to place the body part exactly in the same position inside the coil each time the apparatus is used.

### Summary of the invention

It is an object of the present invention to provide an apparatus for treating a body part of the aforementioned kind, wherein the body part can always be easily placed in the same position.

This object is solved by an apparatus according to claim 1. In said apparatus, supporting structure comprises a rigid receiving surface extending through the coil substantially in parallel to the center axis thereof and being arranged so as to support the body part in a position which is displaced, from the center of the cylindrical coil, in the direction of the inner periphery of the cylindrical coil.

The coil is accommodated in a substantially ring-shaped housing, and the ring-shaped housing is fixed to said supporting structure so that an upper part of said housing extends above said receiving surface, while a lower part of said housing extends below said receiving surface, and wherein the cross-sectional area of the coil in said upper part of said housing is smaller than the cross-sectional area of the coil in the lower part of said housing.

The reason for supporting the body part in this displaced position is as follows. It has been found out that using particular pulse patterns, e.g. a pulse pattern for treating osteoporosis, the electromagnetic field is stronger near the inner periphery of the coil than in the center of the coil. Therefore, the supporting structure according to the present invention is arranged so as to support the body part to be treated in a position where the electromagnetic field is relatively strong. Moreover, as a relative long wave length is used, the transverse components of the electromagnetic field, i.e. the components which are not parallel to the center axis of the coil, are substantially zero in this area of the coil.

Advantageous features of the present invention are disclosed in the dependent claims.

Preferably, the electromagnetic field strength is about 12,5 Gauss, and the pulsing rate is between 10 and 20 pulse bursts per second. It has been found out that these values are particularly suitable to be used in the inventive apparatus.

The pulsed DC voltage comprises time-varying rectangular pulses, at least some of which preferably have different durations and amplitudes. There might, however, sometimes be two or three adjacent pulses having the same duration and/or amplitude. As already pointed out above, the particular pulse pattern to be used in the apparatus according to the present invention depends on the disease or condition to be treated.

Preferably, said cylindrical coil has an inner diameter of about 29,04 cm (11 "). Such an inner diameter may be useful in treatment of the hand or knee. For a treatment of the entire body, an opening of, for example, 58,05 cm (22 ") may be suitable for most patients.

In a particularly preferred embodiment of the invention, the ratio of duty cycle to off cycle of the pulses is more than 1. The off cycle is therefore always shorter than the duty cycle; the ratio might for example be 0,52 (duty cycle) to 0,48 (off cycle).

### Brief description of the drawings

Other features and advantages of the present invention will become readily apparent from the following description taken in conjunction with the accompanying drawings, in which:
- Fig. 1: shows an embodiment of an apparatus for treating a body part according to the present invention,
- Fig. 2: schematically shows the shape of the electromagnetic field which is generated in the coil of the apparatus according to the present invention, and
- Fig. 3: shows a pattern of the voltage to be applied to the coil of the apparatus according to the present invention.

### Detailed description of a preferred embodiment

An embodiment of the present invention will hereinafter be described with reference to the accompanying drawings.

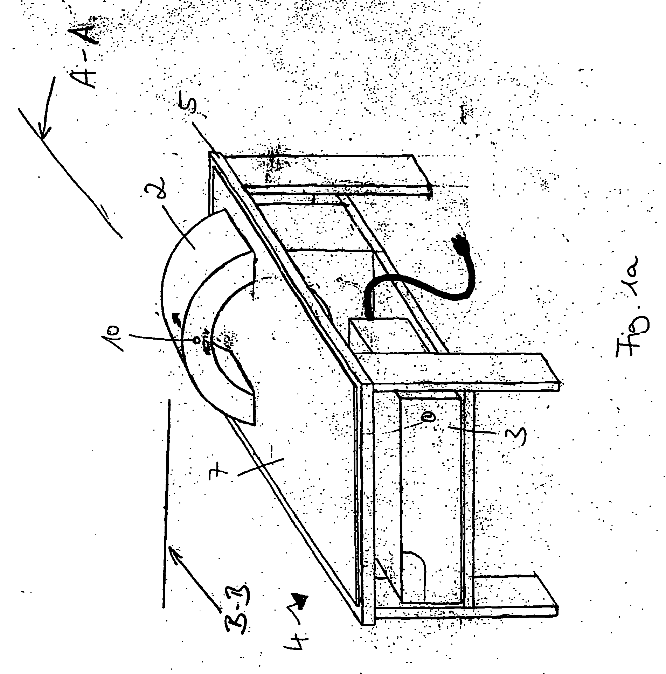

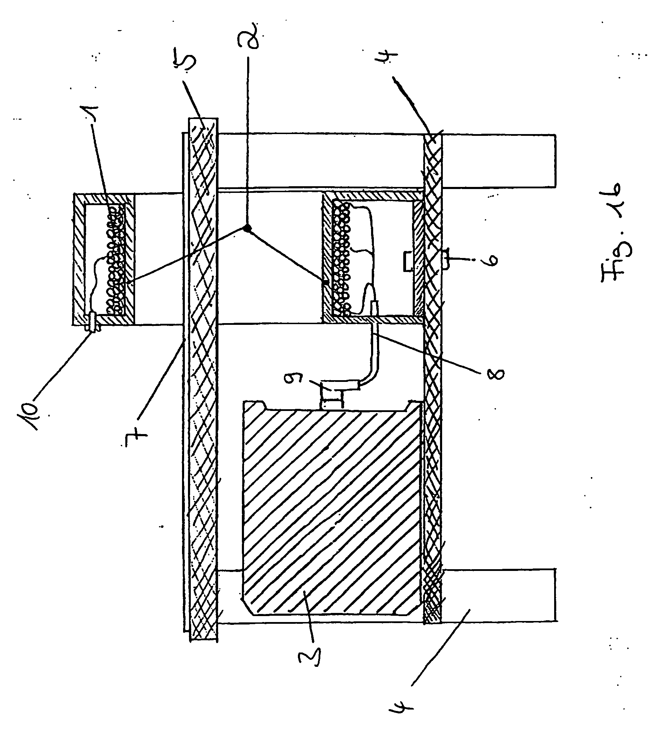

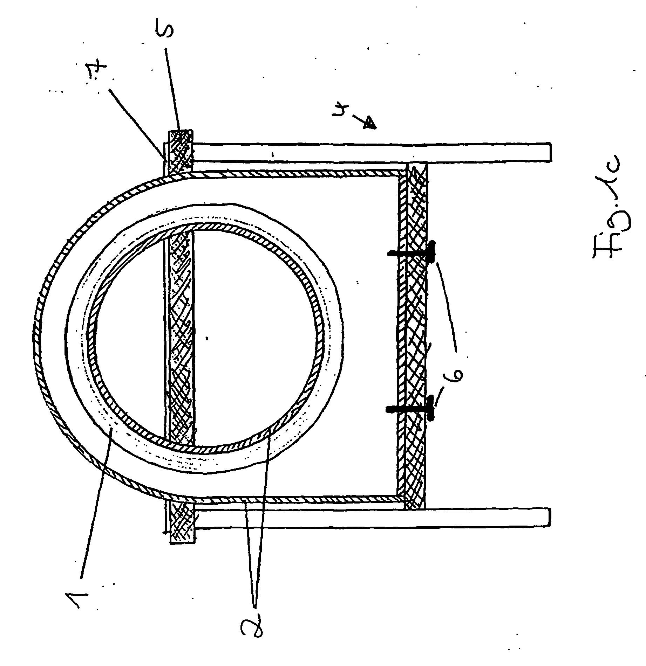

Fig. 1 shows a preferred embodiment of the apparatus for treating a body part according to the present invention. Fig. 1a is a perspective view thereof, Fig. 1b is a cross sectional view along the line A-A in Fig. 1a, and Fig. 1c is a cross sectional view along the line B-B in Fig. 1a.

The apparatus according to this embodiment may be used for treating a patient's arm or leg. The apparatus comprises a cylindrical coil 1 having an inner diameter of preferably about 30 cm. The coil 1 is disposed in a housing 2 which is made of plastic. The housing 2 has the shape of a ring, and its shape is adapted to the shape of the cylindrical coil 1. For energizing the coil C using the above mentioned particular pulse patterns, a control unit 3 is provided, which is connected to the coil 1 via a cable 8 and plug 9. Finally, a LED 10 is provided to indicate an active state of the apparatus.

The apparatus further comprises supporting structure 4 having a rigid receiving surface 5 for supporting the patient's arm or leg (not shown) inside the coil 1. It becomes clear from the Figures that the body part is supported in a position which is displaced, from the center of the cylindrical coil 1, in the direction of the inner periphery of the cylindrical coil 1. In the present embodiment, the coil 1 is positioned upright, and the body part will be displaced upwards relative to the center of the coil 1. In this position, the body part is positioned in an area of the coil 1 where the electromagnetic field is stronger than in the center of the coil 1.

In case a patient's arm is treated with this embodiment of the invention, the patient would preferably sit beneath the apparatus and lay his arm onto the receiving surface 5 so that the arm extends through the opening of the coil 1, substantially in parallel to the center axis of the coil 1.

In the present embodiment, the housing 2 of the coil is fixed to the supporting structure 4 comprising the rigid receiving surface 5 by means of bolts 6. In particular, the receiving surface 5 is arranged relative to the ring-shaped housing 2 so that an upper part of the ring-shaped housing 2 accommodating the coil 1 is positioned above the receiving surface 5, whereas a lower part of the ring-shaped housing 2 is positioned beneath the receiving surface 5. For example, the upper part may be 1/3 of the total cross sectional area of the coil, while the lower part would then be 2/3 of said total cross sectional area.

The supporting structure 4 should be entirely made of a material which does not disturb the electromagnetic field. For example, the structure may be made of wood or plastics. On top of the receiving surface 5, a thin cushioning layer 7 may be provided which may be made of foam rubber.

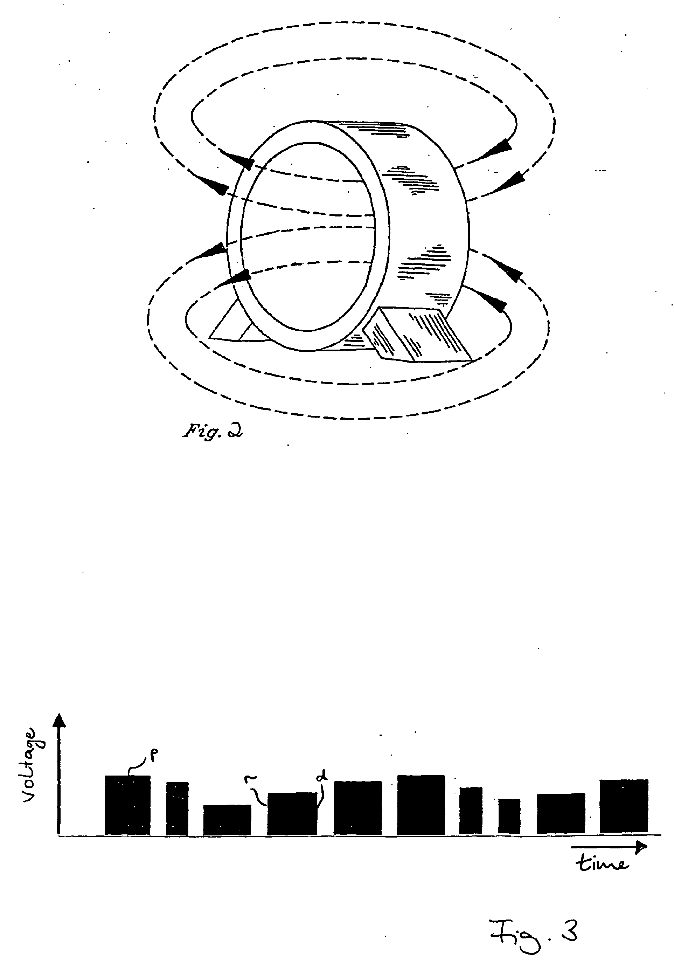

By energizing said cylindrical coil 1, an electromagnetic field is created. Fig. 2 schematically shows the shape of the generated field. It is an important characteristic of the invention that the field not be greater than 20 Gauss in the area of the body part to be treated.

The voltage to be supplied to the coil C has a pattern as for example the one demonstrated in Fig. 3. The voltage supplied must repeatedly build up steeply, hold, and then deteriorate steeply and so that there are thus created a series of spaced working plateaus p of pure DC current. It is preferred, therefore, that in the duty cycle the wave form of the pure DC voltage involved be virtually of rectangular shape with the abruptly rising r and abruptly falling d sides of the wave form comprising sides of a rectangle. In between two such duty cycles there is an off cycle.

As pointed out above, the repetition rate of the pulses corresponds to a rate of 1 to 30 pulse bursts per second. For such a long wave length, the transverse components of the electromagnetic field, i.e. the components which are not parallel to the center axis of the coil, are substantially zero in this area of the coil.

It is moreover important that the movement of the field along the diseased body part be toward its distal end. This is achieved by supplying voltage of proper polarity to the coil leads.

The present invention has been discussed so far by way of one embodiment but should not be construed as being limited to the above embodiment, and it is taken for granted that the above embodiment may be properly changed and modified.

For example, in the above described embodiment, the coil is arranged in an upright position. The coil might, however, be as well arranged in a reclined position, for example for treating a patient's leg while he has put his foot on the floor. In this case, the supporting structure would serve as a kind of abutment for correctly positioning the body part relative to the coil.

Furthermore, the apparatus according to the above described embodiment is constructed and arranged to preferably treat a patient's arm or leg. For treating other body parts as for example the torso, the construction, in particular the size, and the arrangement of the apparatus may be suitably adapted.

A method for treating a body part using the apparatus according to the invention includes the step of generating an electromagnetic field of under 20 Gauss by means of a cylindrical coil being arranged so as to accommodate the body part and to subject said body part to said electromagnetic field. The coil is driven by a pulsed DC voltage having a rectangular wave form consisting of abruptly rising and abruptly deteriorating current pulsing at the rate of 1 to 30 pulse bursts per second. Said body part is support said body part inside said cylindrical coil by means of supporting structure comprising a rigid receiving surface extending through the coil substantially in parallel to the center axis thereof. The body part is supported in a position which is displaced, from the center of the cylindrical coil, in the direction of the inner periphery of the cylindrical coil.

## Claims

1. Apparatus for treating a body part, comprising
- a cylindrical coil (1) for generating an electromagnetic field of under 20 Gauss, the cylindrical coil (1) being arranged so as to accommodate the body part and to subject said body part to said electromagnetic field,
- means for driving the coil by a pulsed DC voltage having a rectangular wave form consisting of rising and lowering the current at the rate of 1 to 30 pulse bursts per second, and
- supporting structure (4) configured to support said body part inside said cylindrical coil (1), wherein said supporting structure (4) comprises a rigid receiving surface (5) extending through the coil (1) substantially parallel to the center axis thereof and being arranged so as to support the body part in a position which is displaced, from the center of the cylindrical coil (1) towards a direction of the inner periphery of the cylindrical coil (1), wherein the coil (1) is accommodated in a substantially ring-shaped housing (2), said ring-shaped housing (2) being fixed to said supporting structure (4) so that an upper part of said housing (2) is positioned above said receiving surface (5) and a lower part of said housing (2) is positioned below said receiving surface (5), **characterized in that** the cross-sectional area of the coil (1) in the upper part is smaller than the cross-sectional area of the coil (1) in the lower part.

2. Apparatus according to claim 1, wherein the electromagnetic field strength is about 12,5 Gauss.

3. Apparatus according to claim 1 or 2, wherein the pulsing rate is between 10 and 20 pulse bursts per second.

4. Apparatus according to any one of the preceding claims, wherein said pulsed DC voltage comprises time-varying rectangular pulses, at least some of which have different durations and amplitudes.

5. Apparatus according to any one of the preceding claims, wherein said cylindrical coil (1) has an inner diameter of about 29,04 cm (11 ").

6. Apparatus according to any one of the preceding claims, wherein ratio of duty cycle to off cycle of the pulses is more than 1.

## Patentansprüche

1. Vorrichtung zur Behandlung eines Körperteils, mit:
- einer zylindrischen Spule (1) zum Erzeugen eines elektromagnetischen Felds von unter 20 Gauss, wobei die zylindrische Spule (1) so angeordnet ist, dass sie den Körperteil aufnimmt und den Körperteil dem elektromagnetischen Feld aussetzt,
- einer Einrichtung zum Antreiben der Spule durch eine gepulste Gleichspannung mit einer rechteckigen Wellenform, die aus einem Anstieg und einer Absenkung des Stroms mit einer Rate von 1 bis 30 Impulssignalen pro Sekunde besteht, und
- einer Stützstruktur (4), die so ausgebildet ist, dass sie den Körperteil im Innern der zylindrischen Spule (1) stützt, wobei die Stützstruktur (4) eine starre Aufnahmeoberfläche (5) umfasst, die sich durch die Spule (1), im Wesentlichen parallel zu der Mittelachse von ihr, erstreckt, und so angeordnet ist, dass sie den Körperteil in einer Position stützt, die von der Mitte der zylindrischen Spule (1) zu einer Richtung des inneren Umfangs der zylindrischen Spule (1) hin verschoben ist, wobei die Spule (1) in einem im Wesentlichen ringförmigen Gehäuse (2) aufgenommen ist, wobei das ringförmige Gehäuse (2) so an der Stützstruktur befestigt ist, dass ein oberer Teil des Gehäuses (2) oberhalb der Aufnahmeoberfläche (5) positioniert ist, und ein unterer Teil des Gehäuses (2) unterhalb der Aufnahmeoberfläche (5) positioniert ist, **dadurch gekennzeichnet, dass** der Querschnittbereich der Spule (1) in dem oberen Teil kleiner als der Querschnittbereich der Spule (1) in dem unteren Teil ist.

2. Vorrichtung nach Anspruch 1, bei der die elektromagnetische Feldstärke ungefähr 12,5 Gauss beträgt.

3. Vorrichtung nach Anspruch 1 oder 2, bei der die Impulsrate zwischen 10 und 20 Impulssignalen pro Sekunde beträgt.

4. Vorrichtung nach einem der vorhergehenden Ansprüche, bei der die gepulste Gleichspannung mit der Zeit variierende rechteckige Impulse umfasst, wobei zumindest einige von ihnen eine unterschiedliche Dauer und Amplitude aufweisen.

5. Vorrichtung nach einem der vorhergehenden Ansprüche, bei der die zylindrische Spule (1) einen Innendurchmesser von ungefähr 29,04 cm (11'') aufweist.

6. Vorrichtung nach einem der vorhergehenden Ansprüche, bei der ein Verhältnis des Arbeitszyklus zu dem Aus-Zyklus der Impulse mehr als eins beträgt.

## Revendications

1. Appareil pour traiter une partie de corps, comprenant
- une bobine cylindrique (1) pour générer un champ électromagnétique de moins de 20 Gauss, la bobine cylindrique (1) étant agencée de manière à recevoir la partie de corps et à soumettre ladite partie de corps audit champ électromagnétique,
- un moyen pour exciter la bobine par une tension d'impulsion CC ayant une forme d'onde rectangulaire consistant en augmenter et baisser le courant au taux de 1 à 30 paquets d'impulsions par seconde, et
- une structure de support (4) configurée de manière à supporter ladite partie de corps dans ladite bobine cylindrique (1), dans lequel ladite structure de support (4) comprend une surface de réception rigide (5) s'étendant à travers la bobine (1) sensiblement parallèlement à l'axe central de celle-ci et étant agencée de manière à supporter la partie de corps dans une position qui est déplacée, du centre de la bobine cylindrique (1) vers une direction de la périphérie intérieure de la bobine cylindrique (1), dans lequel la bobine (1) est reçue dans un logement sensiblement en forme d'anneau (2), ledit logement en forme d'anneau (2) étant fixé sur ladite structure de support (4) de telle manière qu'une partie supérieure dudit logement (2) est positionnée au dessus de ladite surface de réception (5) et qu'une partie inférieure dudit logement (2) est positionnée en dessous de ladite surface de réception (5), **caractérisé en ce que** l'aire de section transversale de la bobine (1) dans la partie supérieure est plus petite que l'aire de section transversale de la bobine (1) dans la partie inférieure.

2. Appareil selon la revendication 1, dans lequel la force du champ électromagnétique est environ 12,5 Gauss.

3. Appareil selon la revendication 1 ou 2, dans lequel le taux d'impulsions est entre 10 et 20 paquets d'impulsions par seconde.

4. Appareil selon l'une quelconque des revendications précédentes, dans lequel ladite tension d'impulsion CC comprend des impulsions rectangulaires variant avec le temps, dont au moins certaines ont des durées et des amplitudes différentes.

5. Appareil selon l'une quelconque des revendications précédentes, dans lequel ladite bobine cylindrique (1) a un diamètre intérieur d'environ 29,04 cm (11 pouces).

6. Appareil selon l'une quelconque des revendications précédentes, dans lequel le rapport du cycle d'utilisation au cycle de non utilisation des impulsions est supérieur à 1.
